# EUROPÄISCHE PATENTSCHRIFT

(11) **EP 0 842 190 B1**
(45) Veröffentlichungstag und Bekanntmachung des Hinweises auf die Patenterteilung: **06.10.1999**
(21) Anmeldenummer: 96925744.3
(22) Anmeldetag: 12.07.1996
(51) Int. Cl.: C07K 7/06, C07K 5/06, C07K 5/08, C07K 5/10

(54) **VERFAHREN ZUR HERSTELLUNG VON DOLASTATIN 15 UND DEREN ZWISCHENPRODUKTEN**
PROCESS FOR PRODUCING DOLASTATIN 15 AND INTERMEDIATES THEREOF
PROCEDE DE PREPARATION DE DOLASTATINE 15 ET SES PRODUITS INTERMEDIAIRES

(30) Priorität: 28.07.1995 DE 19527575
(43) Veröffentlichungstag der Anmeldung: 20.05.1998
(73) Patentinhaber: BASF AKTIENGESELLSCHAFT, 67056 Ludwigshafen (DE)
(72) Erfinder: AMBERG, Wilhelm, D-61381 Friedrichsdorf (DE); BERNARD, Harald, D-67098 Bad Dürkheim (DE); BUSCHMANN, Ernst, D-67069 Ludwigshafen (DE); HAUPT, Andreas, Westborough, MA 01581 (US); JANSSEN, Bernd, D-67063 Ludwigshafen (DE); ULRICH, Karl, D-67061 Ludwigshafen (DE); KLING, Andreas, D-68239 Mannheim (DE); MÜLLER, Stefan, D-67354 Römerberg (DE); RITTER, Kurt, D-69115 Heidelberg (DE); ZIERKE, Thomas, D-67459 Böhl-Iggelheim (DE)
(86) Internationale Anmeldenummer: EP9603073
(87) Internationale Veröffentlichungsnummer: WO9705162

(56) Entgegenhaltungen:
- WO-A-93/23424
- FR-A- 2 460 292
- MONATSHEFTE FUR CHEMIE, Bd. 109, Nr. 1, März 1978, WIEN AT, Seiten 147-155, XP002019748 J HÄUSLER & U SCHMIDT: "Ringschlüsse von Pyruvoylpeptiden und Dehyropeptiden"
- FEBS LETTERS, Bd. 227, Nr. 2, Januar 1988, AMSTERDAM NL, Seiten 171-174, XP002019749 J LASCH ET AL.: "Enzymic properties of intestinal aminopeptidase P: a new continuous assay"
- CHEMICAL ABSTRACTS, vol. 115, no. 25, 23.Dezember 1991 Columbus, Ohio, US; abstract no. 280575j, H KIMURA TE AL.: "Preparation of helodermin fragments as antiasthmatic" Seite 1072; XP002019752 & JP 03 141 298 A (M D RESEARCH K K) 23.Dezember 1991
- Chemical Abstracts, 12th Collective Index, Chemical Substances, Volumes 106-115, Registry Number 137617-30-2 XP002019751 & CHEMICAL ABSTRACTS, vol. 115, no. 25, 23.Dezember 1991 Columbus, Ohio, US; abstract no. 280575j, Seite 1072;
- TETRAHEDRON, Bd. 48, Nr. 20, 15.Mai 1992, OXFORD GB, Seiten 4115-4122, XP002019750 N PATINO ET AL.: "Total synthesis of the proposed structure of Dolastatin 15" in der Anmeldung erwähnt

## Beschreibung

Die Erfindung betrifft ein neues Verfahren zur Herstellung von speziellen Pentapeptiden und die bei der Durchführung des Verfahrens entstehenden neuen Zwischenprodukte.

Dolastatin 15, ein peptidischer Wirkstoff isoliert aus dem Seehasen Dolabella auricularia (G. R. Pettit et al., J. Org. Chem. 1989, 54, 6005), und strukturell verwandte synthetische Peptide, die in WO 93 - 23 424 beschrieben sind, sind vielversprechende neue Wirkstoffe, die sich teilweise bereits in klinischer Prüfung befinden. Da eine Isolierung aus der natürlichen Quelle (6,2 mg aus 1 600 kg Seehase) aussichtslos ist, richtet sich das Interesse auf geeignete synthetische Verfahren, die Wirkstoffe in ausreichender Menge und Reinheit im technischen Maßstab zugänglich machen.

Zwei Verfahren zur Herstellung von Dolastatin 15 sind beschrieben: Die elegante Synthese von G. R. Pettit et al.
(J. Am.Chem.Soc. 1991, 113, 6692 und Tetrahedron 1994, 50, 12097) stellt Dolastatin 15 ausgehend von Prolinmethylester-Hydrochlorid her (Schema 1).

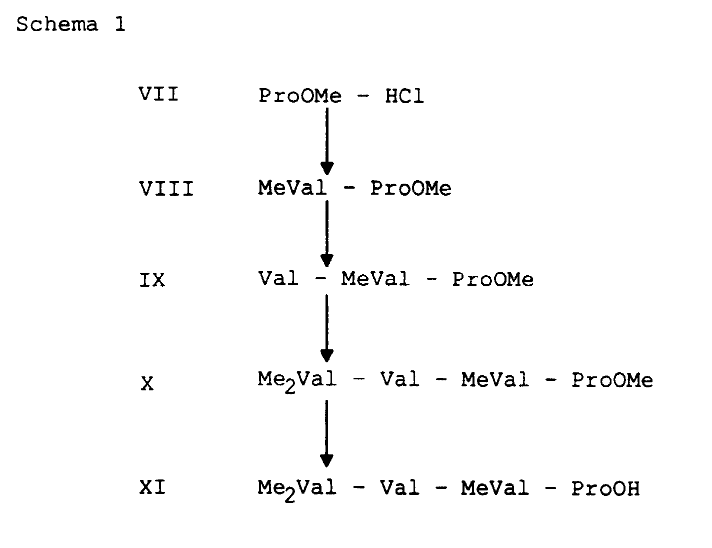

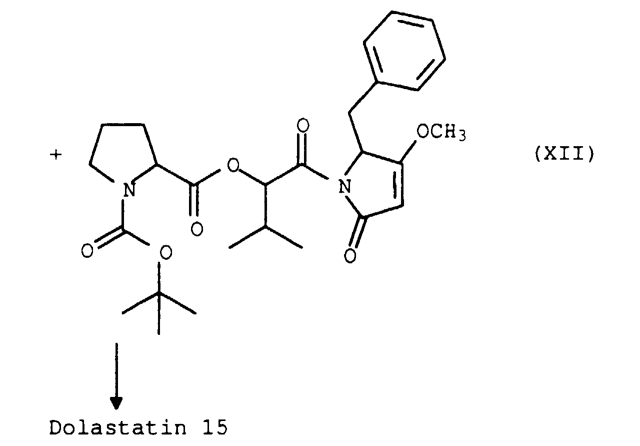

Das oben aufgeführte Verfahren besitzt jedoch die folgenden Nachteile:
1. Die Ausgangsverbindung, Prolinmethylester-Hydrochlorid, ist eine extrem hygroskopische Verbindung. Bei der Herstellung muß sorgfältig unter Feuchtigkeitsausschluß gearbeitet werden, sonst zerfließt das kristalline Material unter teilweiser Esterhydrolyse. Die technische Herstellung ist deshalb erschwert.
2. Das Dipeptid VIII neigt zur Cyclisierung zum Diketopiperazin XIII: 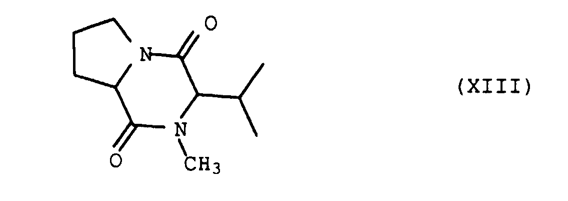 Diese Cyclisierungsreaktion, die im Labor nur zu geringen Ausbeuteverlusten führt, stört bei der Herstellung größerer Substanzmengen im technischen Maßstab beträchtlich.
3. Bei der Herstellung von VIII, IX und X werden jeweils Methylester eingesetzt. Bei den erforderlichen wäßrigen Aufarbeitungen auf diesen Stufen kommt es zur teilweisen Verseifung dieser Ester zu Carbonsäuren. Auch diese Nebenreaktion spielt bei der Maßstabsvergrößerung eine verstärkte Rolle, da sich die Kontaktzeiten des Produktes mit Wasser bei der Reaktionsvergrößerung verlängern.
4. Hydrolyse-empfindlich ist auch der Depsipeptidbaustein XII, der bei der Isolierung und bei der Herstellung des Endproduktes zweimal mit Wasser in Berührung kommt. Die verlängerten Verweilzeiten des technischen Verfahrens führen auch hierbei zu Produktverlusten.

Ein zweites Verfahren zur Herstellung von Dolastatin 15 beschreiben Poncet et al. (Tetrahedron 48, 20, 4115-4112), die vom tert.-Butylester des Prolins ausgehen (Schema 2).

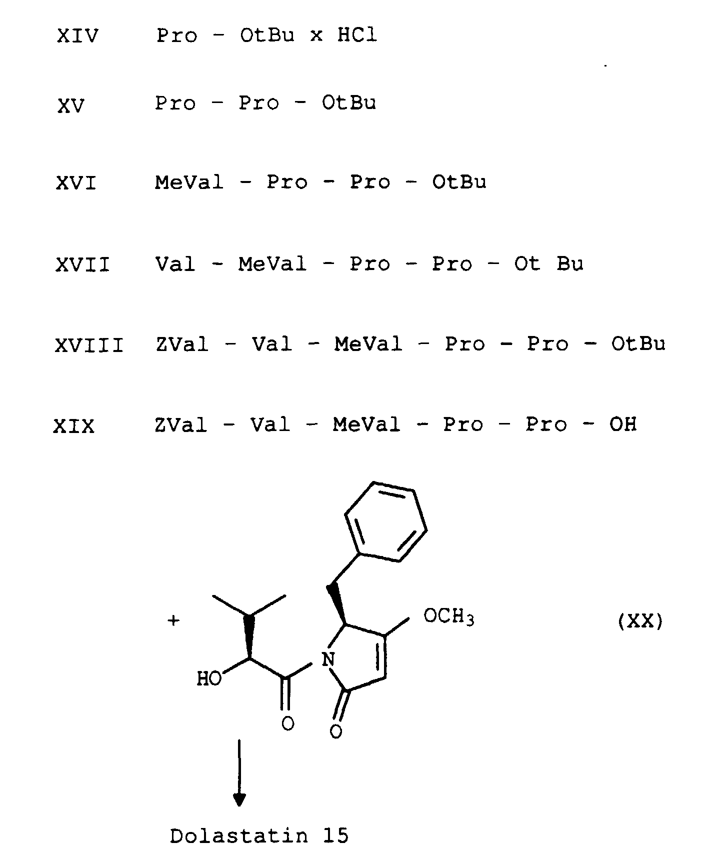

Das Hydrochlorid des Prolin-tert.-butylesters ist weniger hygroskopisch als der nach Schema 1 eingesetzte Methylester. Das Dipeptid XV neigt etwas weniger zur Bildung von Diketopiperazin als der Methylester VIII. Der teure, aufwendig herzustellende Baustein XX wird in einer späteren Synthesestufe eingesetzt, so daß weniger von dieser Verbindung gebraucht wird.

Nachteilig am Verfahren von Poncet (Schema 2) ist, daß der tert.-Butylester mehr Aufwand bei der Herstellung als der Methylester erfordert. Ebenso aufwendig ist die Spaltung dieses Esters mit Trifluoressigsäure, bei der ein brennbares, explosionsfähiges Gas und fluorhaltige, schwierig zu entsorgende Abfälle entstehen.

Nach der Verknüpfung von XIX und XX zu Dolastatin 15 muß zudem noch der Z-Rest gegen zwei Methylgruppen ausgetauscht werden (Schema 3). Bei dieser Operation gehen auf einer späteren Synthesestufe nochmals 20 % des wertvollen Materials verloren.

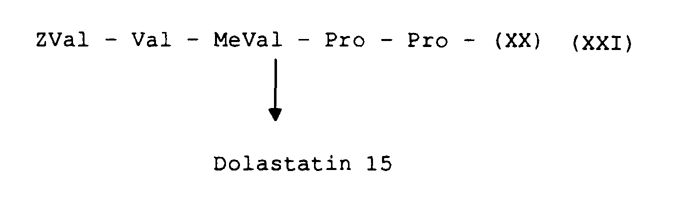
ZVal - Val - MeVal - Pro - Pro - (XX) (XXI)
Dolastatin 15

Die Verfahren von Pettit und Poncet lassen sich auch zur Herstellung zahlreicher Wirkstoffe der WO 93/23.424 nutzen, die strukturell mit Dolastatin 15 verwandt sind. Beispielsweise läßt sich die Tetrapeptidsäure XI mit Prolinbenzylamid zum Wirkstoff Nr. 234 aus WO 93/23.424 verknüpfen (Schema 4).

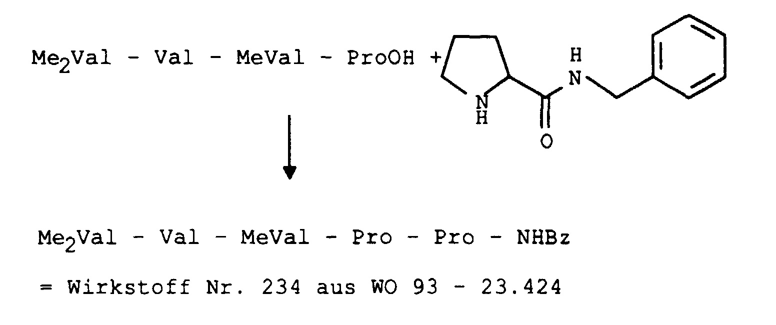

In analoger Weise läßt sich die Pentapeptidsäure XIX aus Schema 2 mit einem Dipeptid zum Heptapeptid XXII umsetzen, aus dem dann Wirkstoff Nr. 1 aus WO 93/23.424 hergestellt werden kann (Schema 5).

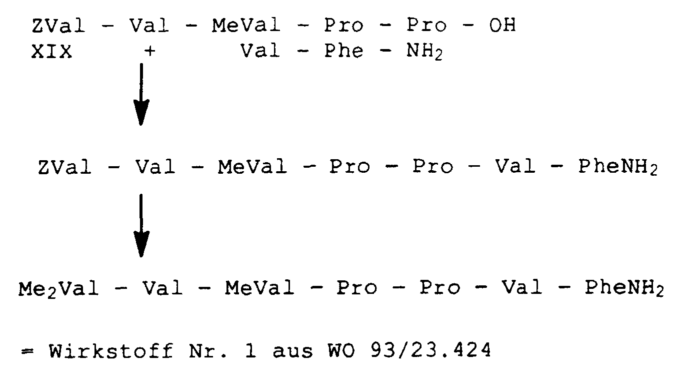
ZVal - Val - MeVal - Pro - Pro - OH
XIX + Val - Phe - NH2
ZVal - Val - MeVal - Pro - Pro - Val - PheNH2
Me2Val - Val - MeVal - Pro - Pro - Val - PheNH2
= Wirkstoff Nr. 1 aus WO 93/23.424

Bei der Wirkstoffherstellung über XIX und XI beeinträchtigen die oben beschriebenen Probleme die Ausbeuten und die technische Durchführbarkeit der Peptidsynthese.

Es wurde nun ein neues Verfahren gefunden, das den Zugang zu den genannten Wirkstoffen erleichtert und auch für die Synthese des Naturstoffes Dolastatin 15 Vereinfachungen bringt.

Gegenstand der Erfindung ist ein Verfahren zur Herstellung von Pentapeptiden der Formel I 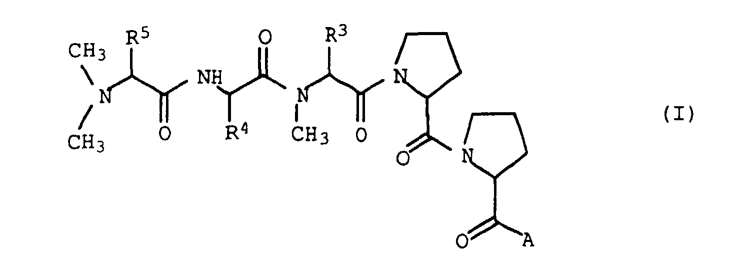 in der
A OH oder eine NR¹R²-Gruppe ist, worin R¹ und R² unabhängig voneinander Wasserstoff, C₁₋₇-Alkyl, Phenyl mit 1, 2 oder 3 Substituenten (unabhängig voneinander C₁₋₆-Alkyl, CF₃, Nitro, Halogen) oder Benzyl mit bis zu drei Substituenten (unabhängig voneinander C₁₋₆-Alkyl, CF₃, Nitro, Halogen) bedeuten,

- R³: für Ethyl, Isopropyl, Isobutyl, tert.-Butyl oder 1-Methylpropyl steht,
- R⁴: für Ethyl, Isopropyl, Isobutyl, tert.-Butyl oder 1-Methylpropyl steht und
- R⁵: für Ethyl, Isopropyl, Isobutyl, tert.-Butyl oder 1-Methylpropyl steht,
welches darin besteht, daß man ein Prolinamid der Formel II 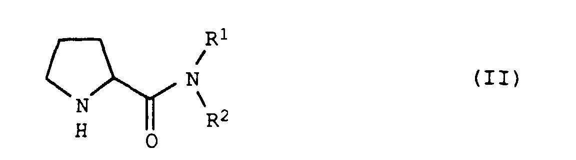 in der R¹ und R² die oben angegebene Bedeutungen haben, in das Dipeptid der Formel III überführt 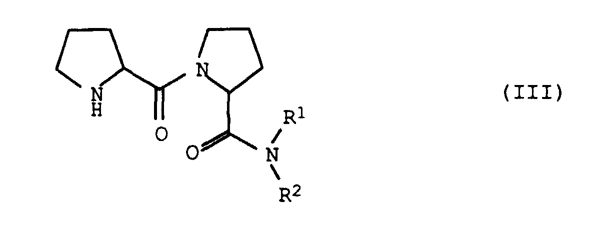 in der R¹ und R² die oben genannten Bedeutungen haben, dieses in das Tripeptid der Formel IV überführt 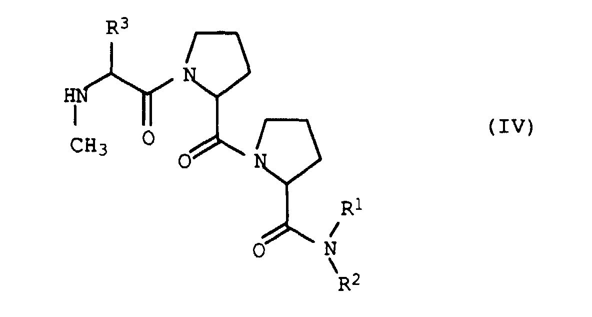 worin R¹, R² und R³ die oben genannten Bedeutungen haben, dieses in das Tetrapeptid der Formel V 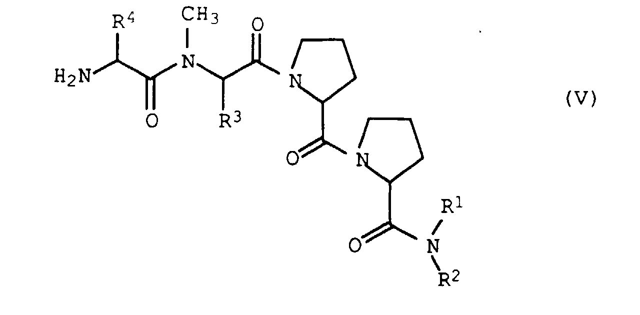 in der R¹, R², R³ und R⁴ die oben genannten Bedeutungen haben, und dieses in das Pentapeptid der Formel I überführt und das so erhaltene Pentapeptid I nach Bedarf mit Prolylendopeptidase (PEP) zur Pentapeptidcarbonsäure VI hydrolysiert

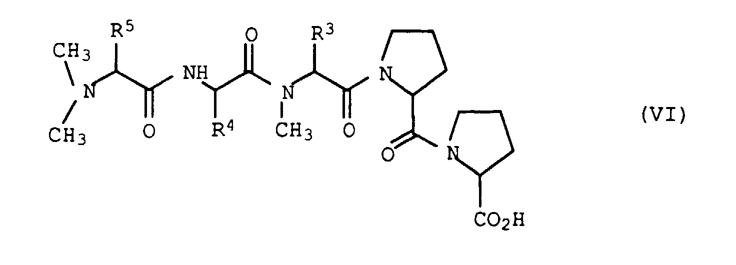

Das Verfahren ist dadurch gekennzeichnet, daß man von Prolinamiden der Formel II ausgeht und daraus über die Peptide III, IV und V zu den Wirkstoffen I gelangt.

Die Verbindungen II, III, IV, V und I können als freie Basen eingesetzt werden. Oft ist es von Vorteil, die Salze dieser Verbindungen mit unterschiedlichen Säuren zu verwenden.

Als Säuren kommen beispielsweise in Frage: HCl, HBr, H₃PO₄, H₂SO₄, Malonsäure, Oxalsäure, Fumarsäure, Maleinsäure, Toluolsulfonsäure, Methansulfonsäure.

Die Verbindungen I und VI sind aus WO 93/23.424 bekannt. Sie stellen wertvolle Peptidwirkstoffe und lassen sich zu anderen Wirksubstanzen weiterverarbeiten, wie beispielsweise zum Naturstoff Dolastatin 15. Dazu wird VI nach den üblichen Methoden aktiviert und wie von Poncet et al. (Tetrahedron 48, 4115 - 4122, 1992) beschrieben mit Baustein XX umgesetzt.

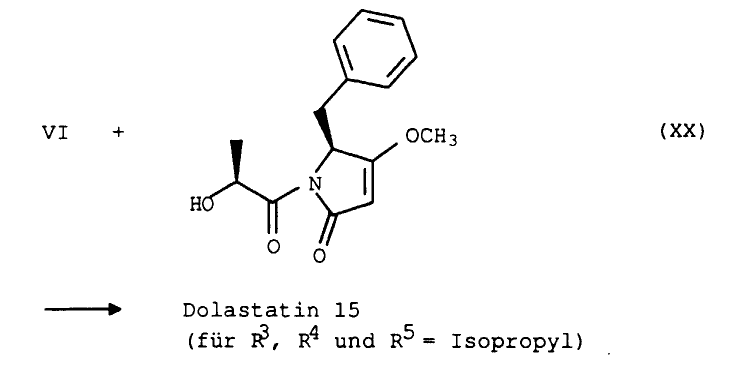

Weitere Wirkstoffe der WO93/23.424 erhält man nach üblichen Methoden der Peptidkopplung aus VI und Aminosäuren bzw. Peptiden.

Die Vorteile des neuen Verfahrens zur Herstellung der genannten Verbindungen und insbesondere des Naturstoffes Dolastatin 15 bestehen im folgenden:

Die Ausgangsverbindungen sind gut zugängliche Prolinamide, die auch in Form ihrer kristallinen Hydrochloride nicht hygroskopisch sind.

Bei der Herstellung des Dipeptides besteht nur in untergeordnetem Maß die Gefahr einer Bildung von Diketopiperazinen. Sowohl beim Verfahren von Pettit als auch bei der Synthesemethode von Poncet muß vor allem bei den längeren Verweilzeiten einer technischen Synthese mit der Bildung von Diketopiperazinen gerechnet werden.

Im Verlauf des neuen Verfahrens ist bei allen Kopplungsstufen die endständige Carboxylgruppe als Amid sehr wirksam geschützt und mit Prolylendopeptidase (PEP) hochspezifisch und einfach abspaltbar. Der von Pettit verwendete Methylester wird hingegen partiell gespalten, während der von Poncet genutzte tert.-Butylester technisch schwieriger herzustellen ist und Probleme bei der Abspaltung bereitet.

Der Baustein XX wird im neuen Verfahren erst in der allerletzten Synthesestufe benötigt, so daß von dieser Substanz weniger benötigt wird als bei dem nur mit großem Aufwand durchführbaren Verfahren von Poncet.

Das erfindungsgemäße Verfahren bietet nicht nur einen guten Zugang zu Dolastatin 15, sondern ermöglicht auch eine sehr praktische Synthese für zahlreiche Wirkstoffe, die in der WO 93/23.424 genannt sind. Die genannte Patentanmeldung beschreibt die Herstellung der antineoplastischen Peptide über eine Festphasensynthese. Diese Methode ist für die Herstellung im technischen Maßstab sehr wenig geeignet.

Die folgenden Beispiele beschreiben das erfindungsgemäße Verfahren.

Prolinamide der Struktur II werden aus Prolinderivaten der Formel XXI hergestellt in der A für einen aktivierenden Substituenten wie Methoxycarbonyl oder Pivaloyl und P für eine Schutzgruppe z. B. tert.-Butoxycarbonyl (BOC) oder Benzyloxycarbonyl (Z) stehen.

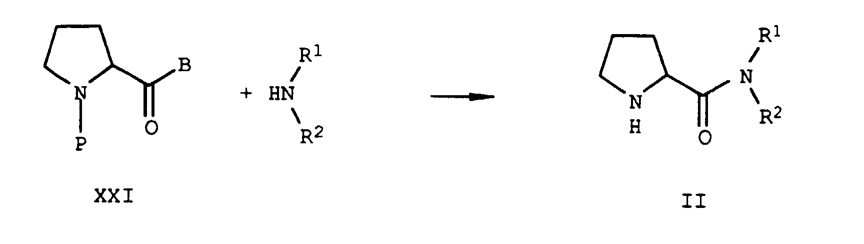

### Beispiel 1: Prolinbenzylamid-Hydrochlorid

Zu einer Lösung von 99,7 g Z-Prolin und 58 ml Triethylamin in 1 l CH₂Cl₂ wurden bei -10°C bis 15°C 48,2 g Pivalinsäurechlorid zugetropft. Man rührte 45 min bei -10°C nach und gab anschließend innerhalb 0,5 h bei -10°C 42,8 g Benzylamin in 500 ml CH₂Cl₂ zu. Man rührte 1 h bei Raumtemperatur nach. Die CH₂Cl₂-Lösung wurde anschließend zweimal mit 500 ml Wasser, zweimal mit 500 ml 10 %iger wäßriger NaHCO₃-Lösung, zweimal mit 500 ml Wasser, zweimal mit 500 ml 5 %iger wäßriger Zitronensäure-Lösung und zweimal mit 500 ml Wasser gewaschen, über Na₂SO₄ getrocknet und eingedampft. Es verblieben 120 g Rückstand, der in 200 ml Essigester aufgenommen wurde. Man gab zur Essigesterlösung 1,2 l n-Heptan zu, rührte 1 h, saugte ab und trocknete bei 50°C im Vakuum. Das so erhaltene Z-Prolinbenzylamid (110 g, Fp. 93-94°C) wurde in 1,5 l Methanol gelöst. Nach Zugabe von 0,5 g Pd/C (10 %ig) wurde Wasserstoff eingeleitet. Die Lösung nahm bei Raumtemperatur innerhalb von 1,5 h 0,5 l H₂ auf. Nach Abfiltrieren des Katalysators und Eindampfen verblieben 4,6 g eines gelben Öls, das ohne weitere Reinigung weiter eingesetzt werden konnte. Hochreines Produkt wurde durch Fällung des Prolinbenzylamid-Hydrochlorids gewonnen. Dazu wurden 4,1 g Prolinbenzylamid in 400 ml Isopropanol gelöst. Man gab 63 ml einer gesättigten Lösung von HCl in Isopropanol zu, rührte die entstandene Suspension 2 h bei 0°C bis -5°C, saugte ab und wusch zweimal mit 250 ml Isopropanol. Der Rückstand wurde bei 50°C im Vakuum getrocknet. Man erhielt 4 g Prolinbenzylamid-Hydrochlorid, [α]²⁰_{D} = -45°C.

Dipeptide der Struktur III werden aus Prolinamiden II und geeigneten Prolinderivaten nach den üblichen Methoden der Peptidkopplung hergestellt.

### Beispiel 2: Pro-Pro-NHBz x HCl

Zu einer Lösung von 249 g Z-Prolin und 202,4 g Triethylamin in 2 l CH₂Cl₂ wurde bei -5°C bis -10°C innerhalb von 20 min 120,6 g Pivalinsäurechlorid zugetropft. Man rührte 60 min bei -55°C und gab anschließend bei -5°C bis -10°C innerhalb von 30 min eine Lösung von 246 g Prolinbenzylamid-Hydrochlorid in 300 ml Methanol zu. Man rührte 1 h bei unter 0°C und 15 h bei Raumtemperatur. Die Lösung wurde gewaschen mit 1 l H₂O, 1 l 10 %iger Essigsäure, 1 l H₂O, 1 l 10 %iger NaOH und 1 l H₂O. Die organische Phase wurde zur Trocknung eingeengt. Der Rückstand wurde in 1 l Methanol aufgenommen und 1 h zum Rückfluß erwärmt. Methanol wurde durch 2,5 1 Isopropanol ersetzt. Nach Zusatz von 30 g Pd/C (5 %ig) wurde 4 h bis zur Sättigung Wasserstoff eingeleitet. Nach Zugabe von 120 ml gesättigter isopropanolischer Lösung von HCl wurde vom Katalysator abfiltriert. Die Mutterlauge wurde eingeengt und der Rückstand mit 400 l Isopropanol versetzt. Nach Beginn der Kristallisation wurden 2 l Methyl-tert.-butylether zugegeben. Man rührte 15 h bei Raumtemperatur, saugte ab, wusch mit Methyl-tert.-butylether und trocknete im Vakuum. Ausbeute: 277 g (81,3 %, Fp. 185,5-187°C, [α]²⁰_{D} = -96°C).

Tripeptide der Struktur IV wurden aus den Dipeptiden III und geeigneten Derivaten von N-Methylaminosäuren (wie z. B. N-Methylvalin, N-Methylleucin, N-Methyl-Isoleucin, N-Methyl-tert.-Leucin) nach den üblichen Methoden der Peptidkopplung hergestellt.

### Beispiel 3: MeVal-Pro-Pro-NHBz x HCl

Zu einer Lösung von 199,5 g Z-MeVal und 253 g Pro-Pro-NHBz x HCl und 417 g Diisopropylethylamin wurde innerhalb von 40 min bei -5 bis -100C 636 g einer 50 %igen Lösung von Propanphosphorsäureanhydrid in Essigester zugetropft. Man rührte 15 h bei Raumtemperatur, gab 1 l Wasser zu und wusch die organische Phase mit 750 ml 10 %iger Essigsäure, 750 ml Wasser, 750 ml 10 %iger NaOH und 750 ml Wasser. Die organische Phase wurde eingeengt und der Rückstand in 2,5 l Isopropanol aufgenommen. Nach Zugabe von 90 g Pd/C (5 %ig) wurde 8 h lang H₂ eingeleitet. Nach Abfiltrieren des Katalysators wurde eingedampft. Der Rückstand wurde in 800 ml Isopropanol gelöst, mit 100 ml Isopropanolischer HCl versetzt und bei 45°C angeimpft. Nach Zugabe von 2 l Methyl-tert.-butylether wurde über Nacht bei Raumtemperatur gerührt, abgesaugt, mit Isopropanol gewaschen und getrocknet. Ausbeute 313 g (92,5 %), Fp. 243-244°C, [α]²⁰_{D} = -141°C.

Tetrapeptide der Struktur V wurden aus den Tripeptiden IV und geeigneten Derivaten der Aminosäuren Valin, α-Aminobuttersäure, Leucin, tert.-Leucin und Isoleucin hergestellt.

### Beispiel 4: Val-MeVal-Pro-Pro-NHBz x HCl

Zu einer Lösung von 188,5 g Z-Valin und 151,2 g Triethylamin in 1,5 l CH₂Cl₂ wurde bei -5°C bis -10°C innerhalb von 20 min 94,5 g Pivalinsäurechlorid zugetropft. Nach 90 min Rühren bei -5 bis -10°C wurde innerhalb von 40 min bei -5°C bis -10°C 337,5 g MeVal-Pro-Pro-NHBz x HCl portionsweise zugegeben. Man rührte 15 h bei Raumtemperatur, setzte 750 ml Wasser zu und wusch die organische Phase mit 750 ml 10 %iger Essigsäure, 750 ml Wasser, 750 ml 10 %iger NaOH und 750 ml Wasser. Die organische Phase wurde eingeengt. Der Rückstand wurde in 2 l Methanol gelöst und 1 h zum Rückfluß erwärmt. Nach Zugabe von 30 g Pd/C (5 %ig), in 60 ml Wasser aufgeschlämmt, wurde bis zur Sättigung (2,5 h) Wasserstoff eingeleitet. Nach Abfiltrieren des Katalysators wurde bis zur Trockne eingeengt. Der Rückstand wurde mit 600 ml Isopropanol versetzt und mit 120 ml 30 %iger isopropanolischer HCl angesäuert. Nach Animpfen wurde 15 h bei Raumtemperatur gerührt. Zum Kristallbrei wurden 2 l Methyl-tert.-butylether zugegeben. Nach 2 h Rühren wurde abgesaugt, mit Isopropanol gewaschen und getrocknet. Man erhielt 306 g (74,1 % Ausbeute), Fp. 205-208, 5°C, [α]²⁰_{D} = -179,5°C.

Pentapeptide der Struktur V wurden aus den Tetrapeptiden und geeigneten Derivaten der Aminosäuren Valin, -Aminoisobuttersäure, Leucin, tert.-Leucin und Isoleucin hergestellt. Die endständigen Methylgruppen konnten nach der Kopplung zum Pentapeptid eingeführt werden. Alternativ können auch geeignete Derivate von N,N-Dimethylvalin, N,N-Dimethyl-aminoisobuttersäure, N,N-Dimethylleucin, N,N-Dimethyl-tert.-Leucin und N,N-Dimethylisoleucin eingesetzt werden.

### Beispiel 5: Me₂Val-Val-MeVal-Pro-Pro- NHBz x HCl (Wirkstoff Nr. 234 aus WO 93/23.424)

Zu einer Lösung von 8,7 g N,N-Dimethylvalin, 27,4 g Val- MeVal-Pro-Pro-NHBz x HCl und 21,6 g Triethylamin in 100 ml CH₂Cl₂ wurden 42,4 g einer 50 %igen Lösung von Propanphosphorsäureanhydrid in Essigester bei 0 bis -6°C innerhalb von 20 min zugetropft. Man rührte 1 h in der Kälte und über Nacht bei Raumtemperatur. Die organische Phase wurde mit 50 ml Wasser gewaschen und eingeengt. Der Rückstand wurde in 50 ml Isopropanol gelöst und mit 10 ml 30 %iger isopropanolischer HCl angesäuert. Man impfte an, setzte bei 60°C 150 ml Methyl-tert.-Butylether zu, rührte über Nacht, saugte ab, wusch mit Isopropanol und trocknete. Man erhielt 29,9 g (88,3 % Ausbeute) [α]²⁰_{D} = -180,3°C.

Die Pentapeptidsäuren VI wurden aus den Pentapeptidamiden V durch Hydrolyse mit Prolylendopeptidase (T. Yoshimoto et al., J. Biol. Chem., 255, 4786 (1980), J. Biochem. 110, 873 (1991)) gewonnen. Besonders geeignete Substrate sind dabei die Benzylamide. Alkylamide lassen sich auch hydrolysieren, reagieren aber wesentlich langsamer.

Die Pentapeptidcarbonsäure VI a = Me₂Val - Val - MeVal - Pro - ProOH ist der geeignete Vorläufer für den Naturstoff Dolastatin 15. Dazu muß VI a mit dem Hydroxysäureamid XX gekoppelt werden. Eine geeignete Synthese für XX beschreiben J. Poncet et al. in Tetrahedron 48 (20) 4115 - 4122 (1992).

### Beispiel 6: Herstellung von Dolastatin 15

Zu einer Lösung von 6 g VI a, 3,0 g XX, 2,0 g DMAP₊ und 2,0 g Triethylamin in 150 ml Methylchlorid wurde bei 0°C eine Lösung von 2,4 g Isopropenylchloroformiat in 20 ml Methylenchlorid zugetropft. Man rührte 15 min bei 0°C und 4 h bei Raumtemperatur. Man wusch mit Wasser, 5 %iger wäßriger NaHCO₃-Lösung und Wasser, trocknete über Na₂SO₄ und engte ein. Der Rückstand wurde in Hexan/ Essigester gelöst und über Kieselgel chromatographiert. Ausbeute: 5,8 g amorpher Feststoff.

## Patentansprüche

1. Verfahren zur Herstellung von Pentapeptiden der Formel I 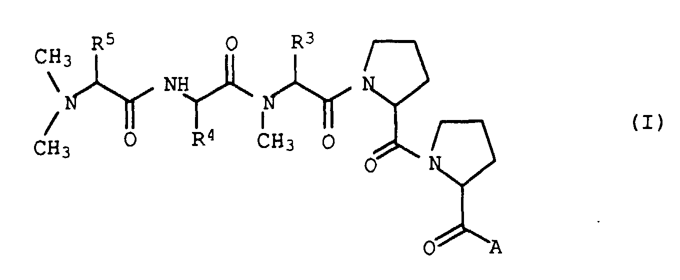 in der
A OH oder eine NR¹R²-Gruppe ist, worin R¹ und R² unabhängig voneinander Wasserstoff, C₁₋₇-Alkyl, Phenyl mit bis zu drei Substituenten (unabhängig voneinander C₁₋₆-Alkyl, CF₃, Nitro, Halogen) oder Benzyl mit bis zu drei Substituenten (unabhängig voneinander C₁₋₆-Alkyl, CF₃, Nitro, Halogen) bedeuten,
R³ für Ethyl, Isopropyl, Isobutyl, tert.-Butyl oder 1-Methylpropyl steht,
R⁴ für Ethyl, Isopropyl, Isobutyl, tert.-Butyl oder 1-Methylpropyl steht und
R⁵ für Ethyl, Isopropyl, Isobutyl, tert.-Butyl oder 1-Methylpropyl steht,
dadurch gekennzeichnet, daß man ein Prolinamid der Formel II 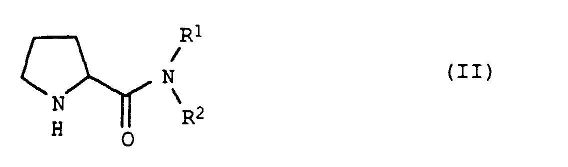 in der R¹ und R² die oben angegebene Bedeutungen haben, in das Dipeptid der Formel III überführt 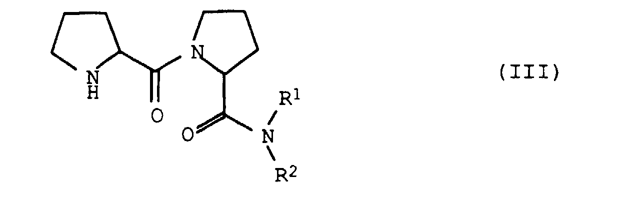 in der R¹ und R² die oben genannten Bedeutungen haben, dieses in das Tripeptid der Formel IV überführt 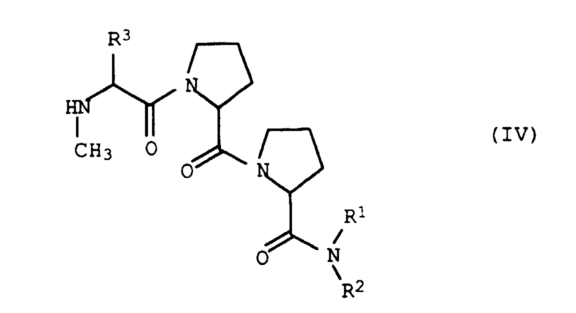 worin R¹, R² und R³ die oben genannten Bedeutungen haben, dieses in das Tetrapeptid der Formel V 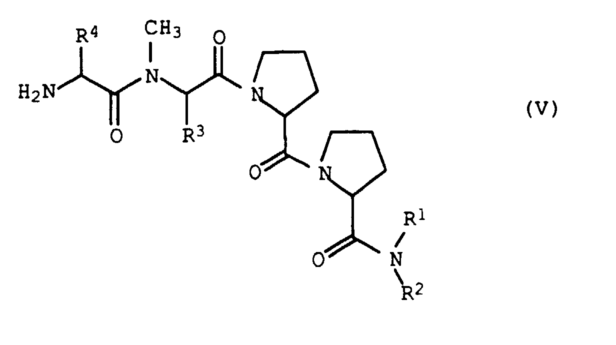 in der R¹, R², R³ und R⁴ die oben genannten Bedeutungen haben, und dieses in das Pentapeptid der Formel I überführt und das so erhaltene Pentapeptid I nach Bedarf mit Prolylendopeptidase (PEP) zur Pentapeptidcarbonsäure VI hydrolysiert

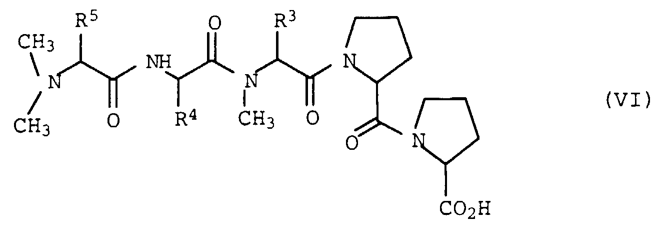

2. Verfahren nach Anspruch 1, dadurch gekennzeichnet, daß man die Substanzen der Formeln II, III, IV, V und I in Form ihrer Salze einsetzt.

3. Vorprodukte zur Herstellung peptidischer Wirkstoffe mit den Formeln IV und V, in denen R¹, R², R³ und R⁴ die oben genannten Bedeutungen haben.

4. Verwendung der Verbindung der Formel VI, worin R³, R⁴ und R⁵ Methylgruppen darstellen, zur Herstellung von Dolastatin 15.

## Claims

1. A process for preparing pentapeptides of the formula I 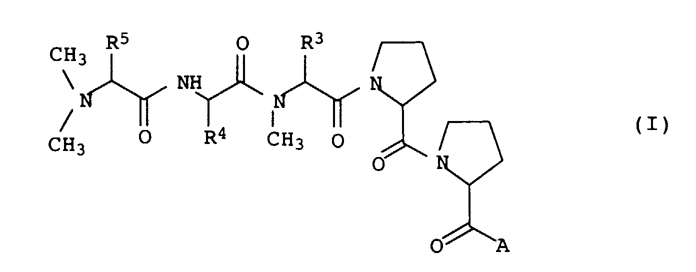 where
A is OH or NR¹R², where R¹ and R² are each, independently of one another, hydrogen, C₁₋₇-alkyl, phenyl with up to three substituents (independently of one another C₁₋₆-alkyl, CF₃, nitro, halogen) or benzyl with up to three substituents (independently of one another C₁₋₆-alkyl, CF₃, nitro, halogen),
R³ is ethyl, isopropyl, isobutyl, tert-butyl or 1-methylpropyl,
R⁴ is ethyl, isopropyl, isobutyl, tert-butyl or 1-methylpropyl, and
R⁵ is ethyl, isopropyl, isobutyl, tert-butyl or 1-methylpropyl,
which comprises converting a prolinamide of the formula II 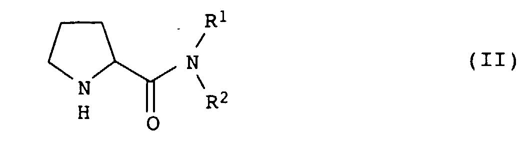 where R¹ and R² have the abovementioned meanings, into the dipeptide of the formula III 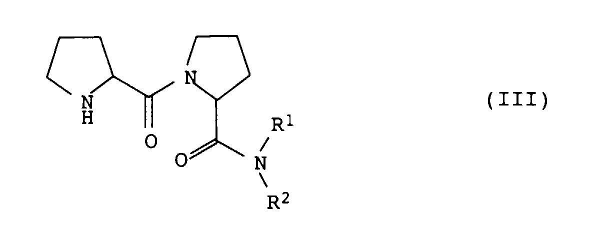 where R¹ and R² have the abovementioned meanings, converting the latter into the tripeptide of the formula IV 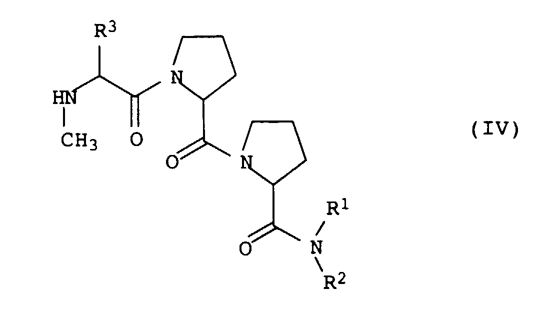 where R¹, R² and R³ have the abovementioned meanings, converting the latter into the tetrapeptide of the formula V 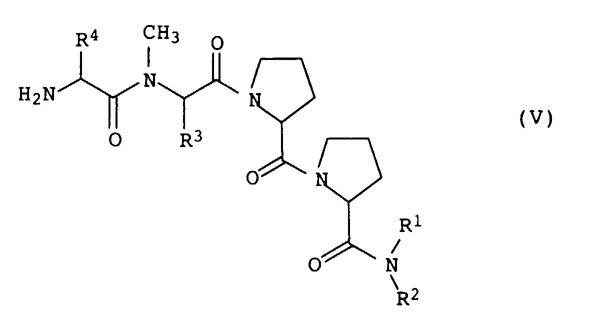 where R¹, R², R³ and R⁴ have the abovementioned meanings, and converting the latter into the pentapeptide of the formula I and, if required, hydrolyzing the resulting pentapeptide I with prolyl endopeptidase (PEP) to give the pentapeptidecarboxylic acid VI

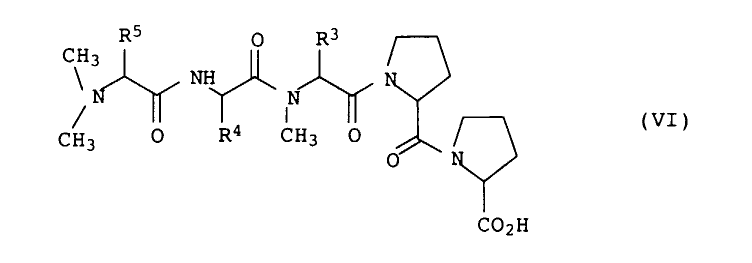

2. A process as claimed in claim 1, wherein the substances of the formulae II, III, IV, V and I are used in the form of their salts.

3. A precursor for preparing active peptides with the formulae IV and V, where R¹, R², R³ and R⁴ have the abovementioned meanings.

4. The use of the compound of the formula VI, where R³, R⁴ and R⁵ are methyl groups, for preparing dolastatin 15.

## Revendications

1. Procédé pour la préparation de pentapeptides de formule I 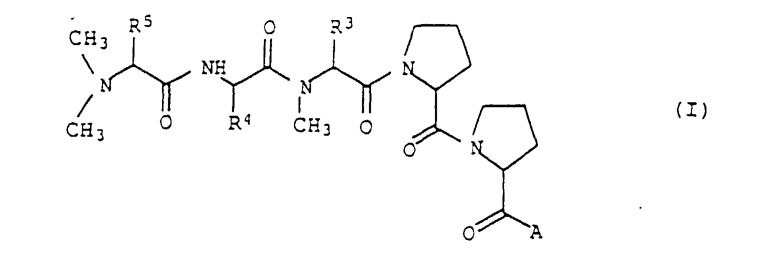 dans laquelle
A représente OH ou un groupe NR¹R² dans lequel R¹ et R² représentent chacun, indépendamment l'un de l'autre, l'hydrogène, un groupe alkyle en C1-C7, phényle portant jusqu'à trois substituants (indépendamment les uns des autres : alkyle en C1-C6, CF₃, nitro, halogéno) ou benzyle portant jusqu'à trois substituants (indépendamment les uns des autres : alkyle en C1-C6, CF₃, nitro, halogéno),
R³ représente un groupe éthyle, isopropyle, isobutyle, tert-butyle ou 1-méthylpropyle,
R⁴ représente un groupe éthyle, isopropyle, isobutyle, tert-butyle ou 1-méthylpropyle et
R⁵ représente un groupe éthyle, isopropyle, isobutyle, tert-butyle ou 1-méthylpropyle, caractérisé par le fait que l'on convertit un prolinamide de formule II 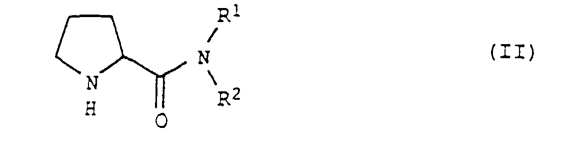
dans laquelle R¹ et R² ont les significations indiquées ci-dessus, en le dipeptide de formule III 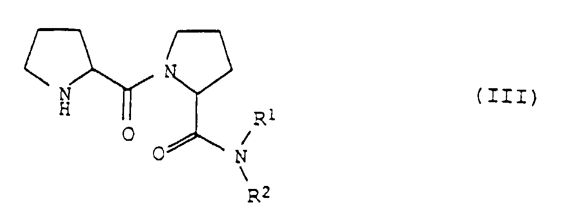 dans laquelle R¹ et R² ont les significations indiquées ci-dessus, on convertit ce dernier en le tripeptide de formule IV 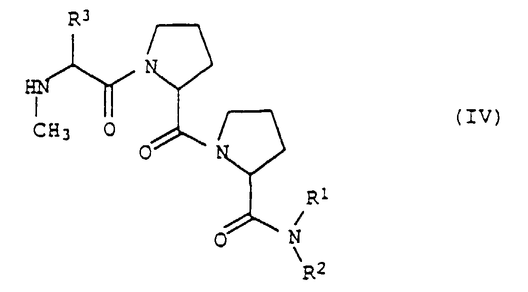 dans laquelle R¹, R² et R³ ont les significations indiquées ci-dessus, on convertit ce dernier en le tétrapeptide de formule V 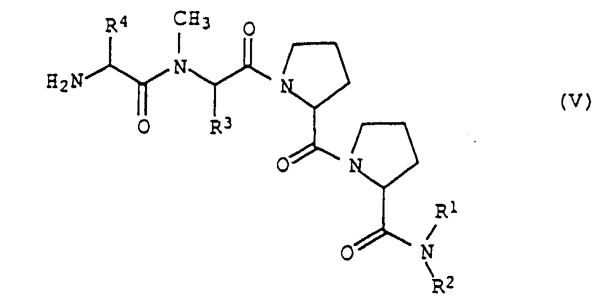 dans laquelle R¹, R², R³ et R⁴ ont les significations indiquées ci-dessus, et on convertit ce dernier en le pentapeptide de formule I que, lorsque c'est nécessaire, on hydrolyse en l'acide pentapeptidecarboxylique VI à l'aide de la prolylendopeptidase (PEP) :

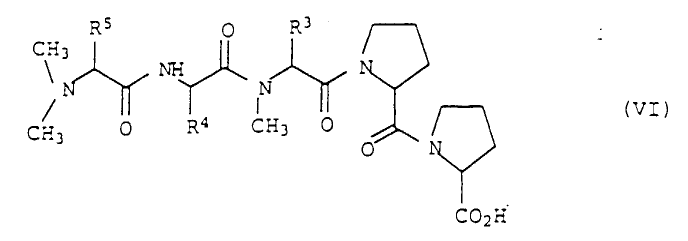

2. Procédé selon la revendication 1, caractérisé par le fait que l'on met en oeuvre les substances de formules II, III, IV, V et I à l'état de sels.

3. Produits intermédiaires de la préparation de substances actives peptidiques, de formules IV et V dans lesquelles R¹, R², R³ et R⁴ ont les significations indiquées ci-dessus.

4. Utilisation du composé de formule VI dans laquelle R³, R⁴ et R⁵ représentent des groupes méthyle pour la prépartion de la dolastatine 15.
